(19) Europäisches Patentamt
European Patent Office
Office européen des brevets

(11) **EP 4 600 236 A1**

(12) **EUROPEAN PATENT APPLICATION**
published in accordance with Art. 153(4) EPC

(43) Date of publication:
**13.08.2025  Bulletin 2025/33**

(21) Application number: **23874877.6**

(22) Date of filing: **03.10.2023**

(51) International Patent Classification (IPC):
***C07C 1/12*** *(2006.01)*  ***C07C 1/04*** *(2006.01)*
***C07C 9/04*** *(2006.01)*

(52) Cooperative Patent Classification (CPC):
**C07C 1/04; C07C 1/12; C07C 1/22; C07C 9/04**

(86) International application number:
**PCT/JP2023/036113**

(87) International publication number:
**WO 2024/075750 (11.04.2024 Gazette 2024/15)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **05.10.2022  JP 2022161211**

(71) Applicant: **DENSO CORPORATION
Kariya-city, Aichi 448-8661 (JP)**

(72) Inventors:
• **KAWADA, Shinji
Kariya- city, Aichi 4488661 (JP)**
• **OKAWA, Hideaki
Kariya- city, Aichi 4488661 (JP)**

(74) Representative: **TBK
Bavariaring 4-6
80336 München (DE)**

(54) **HYDROCARBON GENERATION SYSTEM AND CARBON DIOXIDE CIRCULATION SYSTEM**

(57)    A hydrocarbon generation system (1) includes a hydrocarbon generator (2, 21, 22), an electrolyzer (3), a water vapor supply line (4), and a heat exchanger (51). The hydrocarbon generator generates hydrocarbon through an exothermic reaction between a carbon oxide gas and hydrogen. The electrolyzer generates hydrogen from water vapor of raw materials, the generated hydrogen being supplied to the hydrocarbon generator. The water vapor supply line generates the water vapor of the raw materials by evaporating liquid water of the raw materials and supplies the generated water vapor to the electrolyzer. The heat exchanger uses heat of a reaction generated in the hydrocarbon generator to evaporate the liquid water of the raw materials in the water vapor supply line via heat transfer oil.

FIG.2

EP 4 600 236 A1

## Description

[Cross-Reference to Related Applications]

This international application claims the benefit of priority from Japanese Patent Application No. 2022-161211 filed on October 5, 2022, the entire contents of which are incorporated herein by reference.

[Technical Field]

The present disclosure relates to a hydrocarbon generation system having a hydrocarbon generator and an electrolyzer, and a carbon dioxide circulation system having the hydrocarbon generation system.

[Background Art]

Examples of hydrocarbon generation methods include methanation through the Sabatier reaction, which generates methane from carbon dioxide and hydrogen. Examples of hydrogen generation methods include a technology that generates hydrogen from water vapor of raw materials through an electrolysis reaction.
Patent Literature 1 discloses a method for using hydrogen generated through the electrolysis for methanation. According to the disclosed method in Patent Literature 1, since methanation is an exothermic reaction, heat generated through that reaction is used for hydrogen generation. This method allows for achieving high thermal energy efficiency.

[Citation List]

[Patent Literature]

[PTL 1] JP2019-108238 A

[Summary of the Invention]

In hydrogen generation, heat is required not only for the endothermic reaction through the electrolysis of water vapor but also to generate water vapor itself. Consequently, thermal energy provided by heat generated through the Sabatier reaction is insufficient on its own.
Specifically, as shown in (1) to (3) below, to generate 1 mol of methane through the Sabatier reaction, 4 mol of hydrogen is required. Consequently, 4 mol of liquid water must be converted to water vapor.

$$4H_2O \text{ (liquid)} \rightarrow 4H_2O \text{ (gas)} \ldots \qquad (1)$$

$$4H_2O \text{ (gas)} \rightarrow 4H_2 + 2O_2 \ldots \qquad (2)$$

$$CO_2 + 4H_2 \rightarrow CH_4 + 2H_2O \ldots \qquad (3)$$

For example, an amount of heat required to heat and boil 4 mol of liquid water from 25°C to 100°C, to obtain water vapor (that is, steam) at 100°C, (i.e., the amount of heat necessary for a chemical reaction described in Equation (1)), is 185 kJ. This amount of the heat is greater than 165 kJ of an amount of the heat generated through the Sabatier reaction (i.e., the amount of heat generated through a chemical reaction described in equation (3)). In other words, in principle, the heat of the reaction alone cannot sufficiently provide the heat required to obtain water vapor (i.e., steam) from liquid water. Furthermore, it is challenging to recover 100% of the heat of the reaction, considering heat dissipation; thus, a difference in the amount of the heat becomes even more significant. Therefore, it is essential to supply thermal energy from an external source. An amount of heat generated through a hydrocarbon generation reaction, such as methanation, varies depending on an amount of hydrocarbon generated. Therefore, an amount of heat supplied from external sources must be adjusted depending on the amount of the heat generated through the hydrocarbon generation reaction. However, it is challenging to control the amount of the supplied heat.
The present disclosure is to provide a hydrocarbon generation system capable of generating hydrocarbon while maintaining stable heat exchange, and a carbon dioxide circulation system using the hydrocarbon generation system.
An aspect of the present disclosure provides a hydrocarbon generation system including:

a hydrocarbon generator that generates hydrocarbon through an exothermic reaction between a carbon oxide gas and hydrogen;

an electrolyzer that generates hydrogen from water vapor of raw materials, the generated hydrogen being supplied to the hydrocarbon generator;

a water vapor supply line that generates the water vapor of the raw materials by evaporating liquid water of the raw materials and supplies the generated water vapor to the electrolyzer;

a heat exchanger that uses heat of a reaction generated in the hydrocarbon generator to evaporate the liquid water of the raw materials in the water vapor supply line via heat transfer oil.

Another aspect of the present disclosure provides a carbon dioxide circulation system including:

the hydrocarbon generation system as described above;

a combustion furnace that uses hydrocarbon as fuel and emits carbon dioxide;

a carbon dioxide supply line that supplies carbon dioxide emitted from the combustion furnace to the hydrocarbon generator in the hydrocarbon generation system as the carbon oxide gas; and

a hydrocarbon supply line that supplies hydrocarbon generated by the hydrocarbon generator to the combustion furnace, in which

the carbon dioxide circulation system is configured to adjust an amount of hydrocarbon generated by the hydrocarbon generator depending on an operating status of the combustion furnace.

The hydrocarbon generation system has the hydrocarbon generator, electrolyzer, water vapor supply line, and heat exchanger, as described above. The heat exchanger then uses the heat of the reaction generated in the hydrocarbon generator to evaporate the liquid water of the raw materials in the water vapor supply line. In the heat exchanger, the heat of the reaction is used as thermal energy required for evaporation of the liquid water of the raw materials, via the heat transfer oil.

Heat transfer oil has a high heat capacity, which allows for a higher heat retention after heat recovery in heat exchange. Therefore, if heat generation through the hydrocarbon generation reaction is high, the heat transfer oil recovers sufficient heat of a reaction, which can be used for evaporation of the liquid water of the raw materials in the water vapor supply line. On the other hand, the heat transfer oil can store sufficient heat of a reaction. Therefore, even if an amount of the heat of the reaction generated through the hydrocarbon generation reaction fluctuates and the amount of heat becomes low or zero, heat retained in the heat transfer oil can be applied to heat the liquid water of the raw materials in the water vapor supply line and used as the thermal energy required for evaporation of the liquid water of the raw material. In the hydrocarbon generation system, heat exchange is stable even when the amount of the heat of the reaction fluctuates.

The carbon dioxide circulation system has the hydrocarbon generation system, combustion furnace, carbon dioxide supply line, and hydrocarbon supply line, as described above. **In** the hydrocarbon generator of the hydrocarbon generation system, hydrocarbon is generated through the exothermic reaction between the carbon oxide gas, such as carbon dioxide, and hydrogen. The generated hydrocarbon is supplied to the combustion furnace through the hydrocarbon supply line. **In** the combustion furnace, the combustion of hydrocarbon emits carbon dioxide. The emitted carbon dioxide is supplied to the hydrocarbon generator in the hydrocarbon generation system through the carbon dioxide supply line. Thus, in the carbon dioxide circulation system, carbon dioxide circulates between the hydrocarbon generation system and the combustion furnace. This makes it possible to construct a so-called carbon-neutral system capable of a reduction of greenhouse gas emissions.

The carbon dioxide circulation system is configured so that the amount of hydrocarbon generated by the hydrocarbon generator is adjusted depending on the operating status of the combustion furnace. Accordingly, the hydrocarbon generation system can generate and supply hydrocarbon to the combustion furnace in response to fluctuations in a requirement amount of hydrocarbon, even when the requirement amount thereof fluctuates depending on the operating status of the combustion furnace.

According to the above-described aspects, it is possible to provide a hydrocarbon generation system capable of generating hydrocarbon while ensuring stable heat exchange, and a carbon dioxide circulation system using the hydrocarbon generation system.

Reference signs in parentheses within the claims indicate a correlation with specific means described in the embodiments below and do not limit the technical scope of the present disclosure.

[Brief Description of the Drawings]

The above and other objects, technical features, and advantages of the present disclosure will become clearer with the following detailed description and reference to the accompanying drawings. The drawings are:

FIG. 1 is a block diagram illustrating a main structure of a hydrocarbon generation system according to a first embodiment;

FIG. 2 is a first block diagram illustrating a hydrocarbon generation system that uses carbon dioxide and hydrogen to generate hydrocarbon according to the first embodiment;

FIG. 3 is a second block diagram illustrating a hydrocarbon generation system that uses carbon dioxide and hydrogen to generate hydrocarbon according to the first embodiment;

FIG. 4 is a flowchart illustrating a startup process of the hydrocarbon generation system according to the first embodiment;

FIG. 5 is a flowchart illustrating a hydrogen generation control process of the hydrocarbon generation system according to the first embodiment;

FIG. 6 is a flowchart illustrating a hydrocarbon generation process of the hydrocarbon generation system according to the first embodiment;

FIG. 7 is a flowchart illustrating a temperature control process of the hydrocarbon generation system according to the first embodiment;

FIG. 8 is a block diagram illustrating a hydrocarbon generation system according to a second embodiment;

FIG. 9 is a block diagram illustrating a hydrocarbon generation system according to a third embodiment;

FIG. 10 is an explanatory diagram illustrating a heat balance in the hydrocarbon generation system according to the third embodiment;

FIG. 11 is a graph showing a relationship between a partial pressure of water vapor and a condensation temperature of water according to the third embodiment,

FIG. 12 is a graph showing a relationship between an amount of recovered heat and a temperature of generated gas according to the third embodiment;

FIG. 13 is a graph showing a relationship between a heat exchange area and an amount of recovered condensation heat for each pressure difference of water vapor according to the third embodiment;

FIG. 14 is a graph showing a relationship between a pressure difference of water vapor and a heat exchange area required to recover all condensation heat;

FIG. 15 is a flowchart illustrating a hydrogen generation control process of the hydrocarbon generation system according to the third embodiment;

FIG. 16 is a flowchart illustrating a hydrocarbon generation process of the hydrocarbon generation system according to the third embodiment;

FIG. 17 is a block diagram illustrating a hydrocarbon generation system according to a fourth embodiment; and

FIG. 18 is a block diagram illustrating a hydrocarbon generation system according to a fifth embodiment.

[Description of the Embodiments]

(First Embodiment)

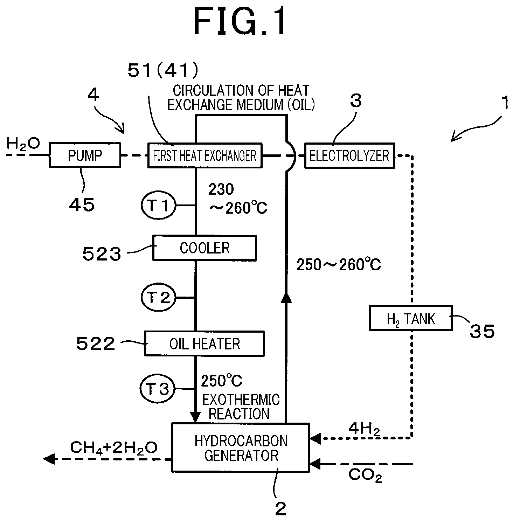

Embodiments for a hydrocarbon generation system 1 are described with reference to FIGs. 1 to 7.

Note that, when "A to B" is used herein to express a range, numerical or physical values indicated in A and B should be regarded as being included in this range. Any numerical or physical value indicated as an upper limit should be regarded as including this value in the upper limit. Any numerical or physical value listed as a lower limit should be regarded as including this value in the lower limit.

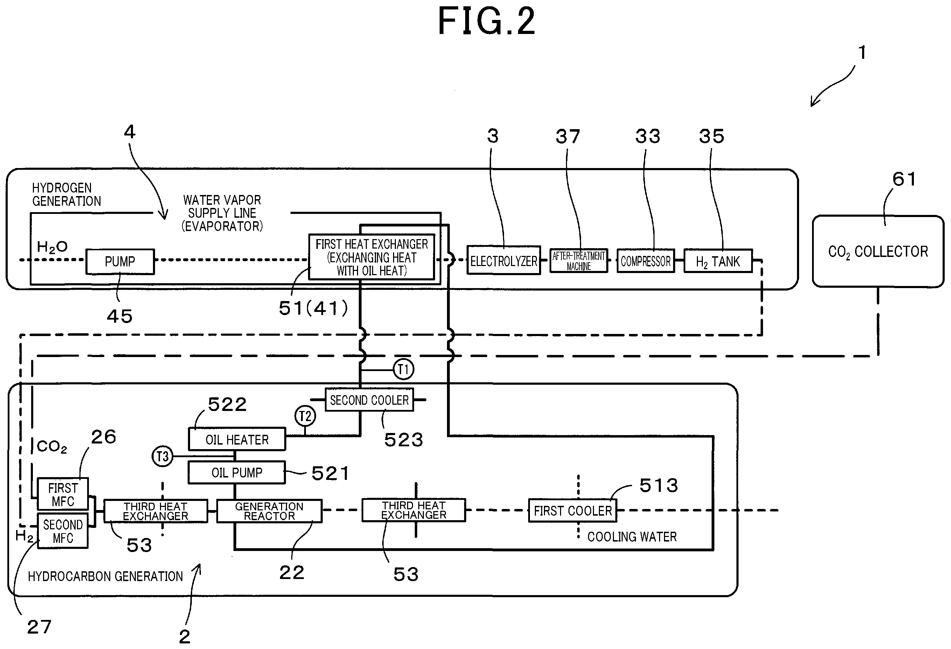

As shown in FIGs. 1 and 2, a hydrocarbon generation system 1 according to a first embodiment is configured to have a hydrocarbon generator 2, an electrolyzer 3, a water vapor supply line 4, and a heat exchanger 51.

The hydrocarbon generator 2 generates hydrocarbon and water vapor through an exothermic reaction between a carbon oxide gas and hydrogen. The hydrocarbon generation reaction in the hydrocarbon generator 2 typically occurs at 200 to 500°C. As a result, gas of 200 to 500°C (i.e., generated gas) is generated.

For example, a carbon oxide gas may include carbon monoxide, carbon dioxide, and the like. The carbon oxide gas may preferably be carbon dioxide in the viewpoint of its easy availability from factory combustion furnaces, the air, and the like. The carbon oxide gas may preferably be carbon monoxide in the viewpoint of its high reactivity in hydrocarbon generation. Carbon dioxide and carbon monoxide may be used together as a carbon oxide gas. For example, the hydrocarbon may include saturated hydrocarbons such as methane and ethane. If the hydrocarbon is methane, the hydrocarbon generation reaction is called methanation. A carbon number of the hydrocarbon can be controlled by changing a catalyst. The hydrocarbon may preferably be methane because it can be used as natural gas in a city (city gas) and can be directly synthesized from carbon dioxide and hydrogen through the Sabatier reaction, as shown in the following equation (I).

The hydrocarbon generation reaction is an exothermic reaction. A chemical reaction equation when a carbon oxide gas is carbon dioxide and hydrocarbon is methane is represented by the following equation (I). "$\Delta H$" in the equation (I) represents an enthalpy change between before and after the chemical reaction.

$$CO_2 + 4H_2 \rightarrow CH_4 + 2H_2O \ (\Delta H = -165 \ kJ/mol) \ ... \qquad (I)$$

As shown in FIG. 2, the hydrocarbon generator 2 specifically has a generation reactor 22 in which the hydrocarbon generation reaction occurs, and first and second mass flow controllers (MFC) 26 and 27 that control an amount of gas supplied to the generator reactor 22. The first mass flow controller 26 controls an amount of a carbon oxide gas, such as $CO_2$, in the supply. The second mass flow controller 27 controls an amount of hydrogen in the supply. The hydrocarbon generation system 1 may further include a $CO_2$ collector 61. The $CO_2$ collector 61 collects and stores carbon dioxide used as a carbon oxide gas from the air, a combustion furnace, or the like. The carbon dioxide is supplied to the electrolyzer 3 and can also be used to generate carbon monoxide.

The electrolyzer 3 generates hydrogen from water vapor (i.e., steam) of raw materials to be supplied to the hydrocarbon generator 2. The electrolyzer 3, a configuration that is not shown in the figure, may include an electrolysis reactor, a cell stack housed in the electrolysis reactor, and the like, for example. A cell stack is composed of multiple solid oxide electrolytic cells (i.e., SOECs). A specific configuration of the electrolyzer 3 can employ a configuration used in water vapor electrolysis using the SOECs and the co-electrolysis of water vapor and carbon dioxide described below. In the electrolyzer 3, an electrolysis reaction of water occurs in the cell stack, as shown in the following equation (II), and hydrogen is generated.

$$H_2O(g) \rightarrow H_2 + 1/2O_2 \ ... \qquad (II)$$

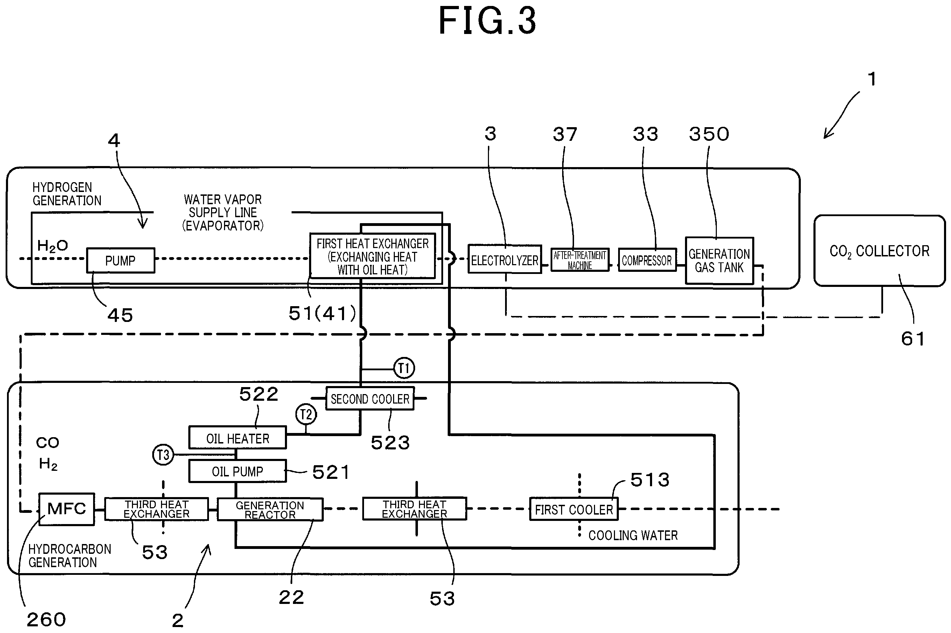

In the electrolyzer 3, carbon monoxide can also be generated along with hydrogen by co-electrolysis with water vapor and carbon dioxide, as shown in the following equation (III). In this case, as shown in FIG. 3, $CO_2$ collected by the $CO_2$ collector 61 is supplied to the electrolyzer 3, and $CO_2$ is electrolyzed in the electrolyzer 3 together with water vapor supplied from the water vapor supply line 4 to generate carbon monoxide along with hydrogen. In the co-electrolysis, carbon monoxide is supplied together with hydrogen to the generation reactor 22 of the hydrocarbon generator 2, and methane can be generated by reacting carbon monoxide and hydrogen in the generation reactor 22, as shown in the following equation (IV). In this case, highly reactive carbon monoxide is used as a carbon oxide gas, which facilitates the hydrocarbon generation reaction. From this perspective, it is preferable to co-electrolyze water vapor and carbon dioxide in the electrolyzer 3. Co-electrolysis can also generate hydrocarbons other than methane, as shown in the following equation (V). 'g' in parentheses in the equations (II) to (V) indicates that a state of matter is gas, i.e., water is water vapor. 'n' in the equation (V) indicates a natural number. Although FIG. 3 shows a configuration in which hydrogen and carbon monoxide are stored in a generation gas tank 350; it is also possible to store hydrogen and carbon monoxide generated by the electrolyzer 3 in separate tanks.

$$CO_2 + 3H_2O(g) \rightarrow CO + 3H_2 + 2O_2 ... \qquad (III)$$

$$CO + 3H_2 \rightarrow CH_4 + H_2O \ (g) \ ... \qquad (IV)$$

$$nCO + (2n+1)H_2 \rightarrow C_nH_{2n+2} + nH_2O(g) \ ... \qquad (V)$$

The water vapor supply line 4 evaporates liquid water of raw materials to generate the water vapor as raw materials, as shown in the following equation (VI), and supplies the generated water vapor to the electrolyzer 3. In the water vapor supply line 4, the liquid water of the raw materials is discharged toward the electrolyzer 3 by a pump 45, for example. The water vapor supply line 4 has an evaporator 41; the evaporator 41 applies thermal energy to heat the liquid water of the raw materials to evaporate this liquid water to generate the water vapor as the raw materials. The pump 45 emits the generated water vapor of the raw materials to the electrolyzer 3. The evaporator 41 is configured to exchange heat with a first heat exchanger 51.

$$H_2O(l) \rightarrow H_2O(g) \ ... \qquad (VI)$$

As shown in FIGs 1 and 2, the heat exchanger 51 (specifically, the first heat exchanger 51) uses the heat of the reaction generated in the hydrocarbon generator 2 for evaporation of the liquid water of the raw materials in the water vapor supply line 4 through heat exchange. The first heat exchanger 51 can recover the generated heat of the reaction via heat transfer oil as a heat exchange medium and apply the recovered heat to heat the liquid water of the raw materials.

For the use of heat transfer oil, an oil heater 522, an oil pump 521, and the like are connected to the first heat exchanger 51, for example. The oil pump 521 is also referred to as a heat transfer oil pump 521. The first heat exchanger 51 can be connected to a cooler 523 (specifically, a second cooler 523) to compensate for a lack of cooling. Note that a refrigerant used in the cooler 523 is water, for example. In FIGs 1 and 2, a flow of the heat transfer oil is represented by a solid line, and a flow direction is counterclockwise. The same is true for the flow of the heat transfer oil illustrated in the subsequent drawings.

No water evaporation means is preferably provided between the first heat exchanger 51 and the electrolyzer 3 in the steam supply line 4. In this case, the liquid water of the raw materials supplied to the first heat exchanger 51 is all vaporized by the heat applied from the heat transfer oil. Therefore, regardless of an amount of heat generated by the hydrocarbon generator 2, the heat applied from the first heat exchanger 51 can generate water vapor as the raw materials from the liquid water of the raw materials. For example, the water evaporation means may include an evaporator similar in configuration to the first evaporator 41, which exchanges heat with the first heat exchanger 51. Since no additional evaporators are required, the hydrocarbon generation system 1 is simplified and easier to control.

The hydrocarbon generation system 1 preferably has a compressor 33 that compresses hydrogen generated by the electrolyzer 3. In this case, the hydrogen pressurized by compression can be supplied to the hydrocarbon generator 2, thus improving reaction efficiency of the hydrocarbon generation reaction in the hydrocarbon generator 2.

Because a response rate of the electrolyzer 3 generally differs from that of the hydrocarbon generator 2, attempts to match both responsiveness will increase a load on either device 2 or 3. Consequently, hydrogen and hydrocarbon generations become unstable, leading to instability in system operations. To avoid this, it is preferable for the hydrocarbon generation system 1 to include a buffer tank 35 (specifically, a hydrogen tank, i.e., an $H_2$ tank 35) for storing hydrogen, disposed between the hydrocarbon generator 2 and the electrolyzer 3. In this case, the buffer tank 35 can overcome differences in an amount of hydrogen transferred due to differences in the response rate, improving operational stability of the system. To enhance this effect, it is preferable to adjust a flow rate of the heat transfer oil to be 50 to 150 times greater than a flow rate of the liquid water or water vapor of the raw materials.

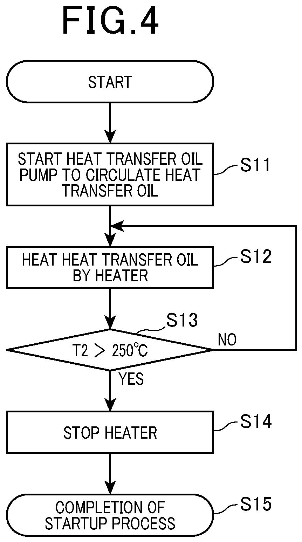

Flowcharts shown in FIGs. 4 to 7 illustrate processes of the hydrocarbon generation system 1 of the present embodiment. In the following description, "T1", "T2", and "T3" refer to respective temperature sensors shown in FIG. 2. For example, the temperature sensor may be a thermometer. The flowcharts are an example of controlling a temperature of the heat transfer oil to be in a range of 240 to 260°C. This temperature may be changed. For example, each process shown in the flowcharts is controlled by a control device that is not shown. Specifically, the control device, which includes one or more processors or the like, controls each process by the processor executing a program stored in a memory.

<Startup Process>

As shown in FIG. 4, when the hydrocarbon generation system 1 begins operations, the heat transfer oil pump 521 starts to circulate the heat transfer oil (S11). The oil heater 522 starts a heating process with the heat transfer oil (S12) and stops the heating process when a temperature of the temperature sensor T2 exceeds 250°C (S13: YES and S14).

<Control of Hydrogen Generation>

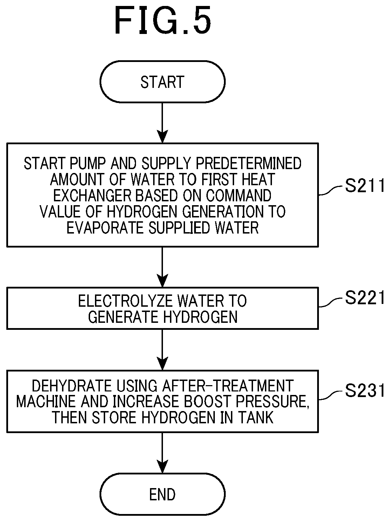

As shown in FIG. 5, when a hydrogen-generating process starts, the pump 45 of the water vapor supply line 4 is activated (S211). The pump 45 discharges a predetermined amount of water (specifically, liquid water of raw materials) to the first heat exchanger 51 based on a command value that defines an amount of hydrogen generated in response to the requirement. In the first heat exchanger 51, water evaporates to generate water vapor of raw materials (i.e., steam), and then the generated water vapor is emitted to the electrolyzer 3. In the electrolysis unit 3, water included in the water vapor is electrolyzed to generate hydrogen (S221). The generated hydrogen is dehydrated by an after-treatment machine 37, compressed by the compressor 33 to increase boost pressure, and stored in the $H_2$ tank 35 (S231) (for example, see FIG. 2).

<Control of Hydrocarbon Generation>

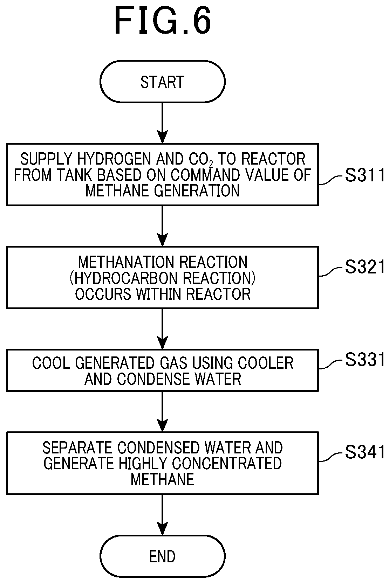

As shown in FIG. 6, when a hydrocarbon-generating process starts, hydrogen and $CO_2$ are supplied to the generation reactor 22 (S311) based on a command value that defines an amount of hydrocarbon generated in response to the requirement (e.g., an amount of methane generated in response to the requirement). The hydrogen is supplied from the $H_2$ tank 35 and $CO_2$ is supplied from the $CO_2$ collector 61. In the generation reactor 22, for example, a methanation reaction occurs to generate a gas containing methane and water (i.e., steam) (S321). The generated gas is supplied to the first heat exchanger 51 to cool and condense the generated water (S331). For example, the condensed water is separated by a gas-liquid separator to generate highly concentrated methane (S341).

<Temperature Control for Heat Transfer Oil>

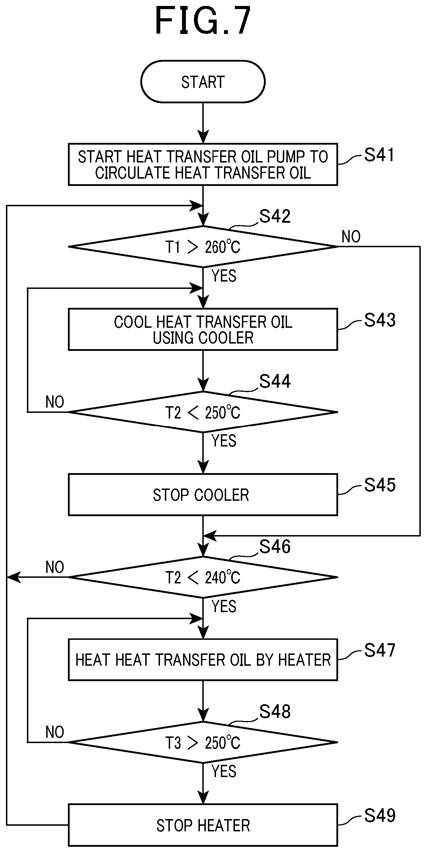

As shown in FIG. 7, a temperature control process for the heat transfer oil is performed by controlling cooling steps S41 to S45 and heating steps S46 to S49 with the heat transfer oil circulated by the heat transfer oil pump 521. In the cooling steps S41 to S45, a temperature determination step S42 of the temperature sensor T1 and a temperature determination step S44 of the temperature sensor T2 (i.e., temperature determination step during cooling) are performed. In the temperature determination step S42, a temperature of the temperature sensor T1 is determined. When the temperature of the temperature sensor T1 exceeds 260°C, for example, the second cooler 523 is activated, and then the heat transfer oil is cooled by the refrigerant (S43). In the temperature determination step S44, a temperature of temperature sensor T2 is determined. When the temperature of the temperature sensor T2 is less than 250°C, for example, the second cooler 523 is stopped (S45). On the other hand, in the temperature determination step S42, when the temperature of the temperature sensor T1 is less than 260°C, for example, the heating steps S46 to S49 are performed. In the temperature determination step S44, when the temperature of the temperature sensor T2 is higher than or equal to 250°C, for example, the heat transfer oil is cooled by the refrigerant in the second cooler 523. Subsequently, in the heating steps S46 to S46, a temperature determination step S46 of the temperature sensor T2 (i.e., temperature determination step during heating) and a temperature determination step S48 of the temperature sensor T3 are performed. In the temperature determination step S46, the temperature of the temperature sensor T2 is determined. When the temperature of temperature sensor T2 is less than 240°C, for example, the oil heater 522 is activated to heat the heat transfer oil (S47). In the temperature determination step S48, when a temperature of the temperature sensor T3 exceeds 250°C, for example, the oil heater 522 is stopped (S49). After the oil heater 522 is stopped, the temperature determination step S42 of the temperature sensor T2 is performed in the cooling process. On the other hand, in the temperature determination step S46, when the temperature of the temperature sensor T2 is higher than or equal to 240°C, for example, the temperature determination step S42 of the temperature sensor T2 is performed in the cooling process. In the temperature determination step S48, when the temperature of the temperature sensor T3 is lower than or equal to 250°C, for example, the oil heater 522 heats the heat transfer oil (S47). Although FIG. 7 shows a process flow in which the cooling steps are performed before the heating steps, the order in which the cooling and heating steps are performed can be interchanged.

In reference to FIG. 1, the temperature control process for the heat transfer oil will be explained in relation to controlling a temperature of the generation reactor 22 to, for example, 250°C.

(a) When the temperature of the temperature sensor T1 is within a range of 240 to 260°C (i.e., an amount of heat generated = an amount of heat recovered), both the cooler 523 and the oil heater 522 are stopped.

(b) When the temperature of the temperature sensor T1 is less than 240°C (i.e., the amount of heat generated < the amount of heat recovered), the cooler 523 is stopped and heating by the oil heater 522 is controlled so that the temperature of temperature sensor T3 reaches 250°C.

(c) When the temperature of the temperature sensor T1 exceeds 260°C (i.e., the amount of heat generated > the amount of heat recovered), the oil heater 522 is stopped and cooling by cooler 523 is controlled so that the temperature of temperature sensor T2 reaches 250°C.

In the Sabatier reaction, when an amount of methane generated by the hydrocarbon generator 2 is 1/4 mol for every 1 mol of hydrogen generated by the electrolyzer 3, this corresponds to the process described in (b) above. However, when water evaporation is stopped for some reason, this may correspond to the process described in (c) above. Therefore, it is preferable to provide the cooler 523 to cool the heat transfer oil. When the amount of heat generated is less than the amount of heat recovered, the temperature of the heat transfer oil decreases gradually. However, using the oil heater 522 ensures that the temperature of the heat transfer oil remains constant throughout a circulation path and keeps the temperature of the generation reactor 22 at a predetermined temperature, such as 250°C.

As shown in FIGs. 1 to 7, the hydrocarbon generation system 1 of the present embodiment is configured to have the hydrocarbon generator 2, electrolyzer 3, water vapor supply line 4, and heat exchanger 51 (specifically, the first heat exchanger 51), as described above. The first heat exchanger 51 uses the heat of the reaction generated by the hydrocarbon generator 2 to evaporate the liquid water of the raw materials in the water vapor supply line 4. In the first heat exchanger 51, the heat of the reaction is used as thermal energy required for evaporation of the liquid water of the raw materials, via the heat transfer oil.

The heat transfer oil has a higher heat capacity than the gas, such as water vapor. Therefore, the heat transfer oil has a higher heat retention after heat recovery. For example, if the amount of heat generated by the hydrocarbon generator 2 is high, the heat transfer oil recovers sufficient heat of a reaction, which can be used for evaporation of the liquid water of the raw materials in the water vapor supply line 4.

On the other hand, the heat transfer oil can store sufficient heat of a reaction. Therefore, even if the amount of the heat of the reaction generated by the hydrocarbon generator 2 fluctuates and the amount of heat becomes low or zero, heat retained in the heat transfer oil can be applied to heat the liquid water of the raw materials in the water vapor supply line 4 and used as the thermal energy required for evaporation of the liquid water of the raw material. This means that a system is highly robust to fluctuations of the heat generation in the hydrocarbon generator 2.

The hydrocarbon generation system 1 has the temperature sensor T2, which measures the temperature of the heat transfer oil flowing into the oil heater 522. The oil heater 522 is preferably disposed downstream of the first heat exchanger 51 in the flow direction of the heat transfer oil in the circulation path of the heat transfer oil, and is configured to control an amount of heating the heat transfer oil based on a measurement value from the temperature sensor T2. In this case, the temperature control of the heat transfer oil is easier. The oil heater 522 is also preferable to be used for raising a temperature of the hydrocarbon generator 2 (i.e., warm-up) during a startup of the hydrocarbon generation system 1. Therefore, if the oil heater 522 is provided in the system, a separate heater will not be needed for the startup.

The hydrocarbon generation system 1 has the cooler 523 (specifically, the second cooler 523), which is disposed downstream of the first heat exchanger 51 in the flow direction of the heat transfer oil in the circulation path of the heat transfer oil and the temperature sensor T1, which measures the temperature of the heat transfer oil flowing into the cooler 523. The cooler 523 is preferably configured to cool the heat transfer oil based on the measurement value from the temperature sensor T1. In this case, for example, as shown in FIG. 7, the cooler 523 cools the heat transfer oil based on the measurement value from the temperature sensor T1 (i.e., a detected temperature). This simplifies controlling the temperature of the heat transfer oil to ensure it stays within a desired range.

(Second Embodiment)

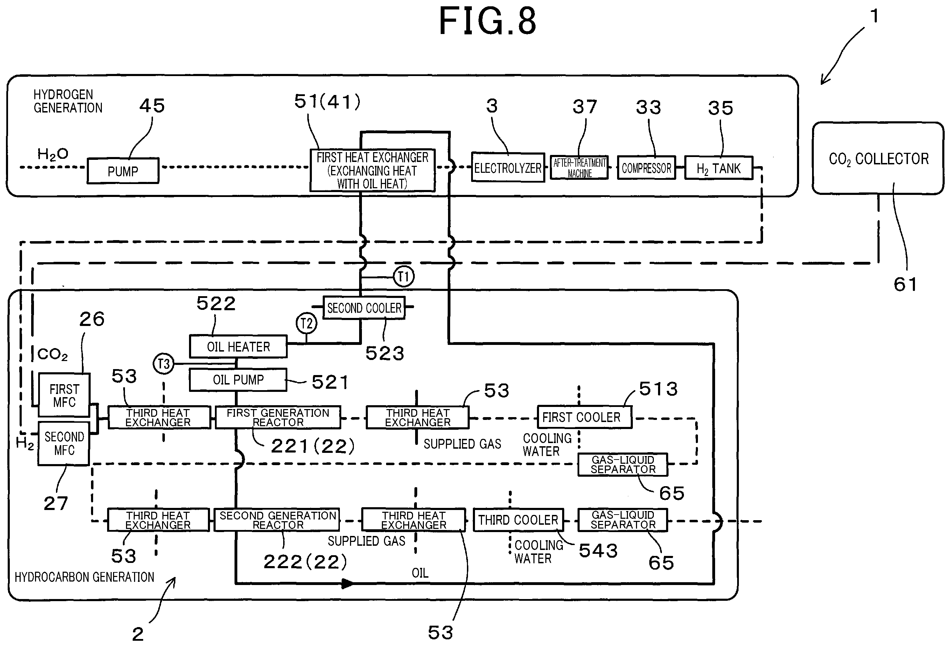

The present embodiment describes an embodiment of a hydrocarbon generation system having two hydrocarbon generators, with reference to FIG 8. In the present embodiment and subsequent embodiments, the same reference signs used in previous embodiments represent the same elements as those in earlier embodiments, unless otherwise specified.

As shown in FIG. 8, a hydrocarbon generation system 1 of the present embodiment has two hydrocarbon generators 2, which are connected in series. Gas flow paths of each hydrocarbon generator 2 are specifically connected in series. The two hydrocarbon generators 2 each have hydrocarbon generation reactors 22. In FIG. 8, a first generation reactor 221 is disposed upstream in the gas flow path, and a second generation reactor 222 is disposed downstream of the first generation reactor 221. Each hydrocarbon generator 2 has a similar configuration to each other, and furthermore, each hydrocarbon generator 2 has a similar configuration to the hydrocarbon generator described in the first embodiment. A third cooler 543 cools the generated gas emitted from the second generation reactor 222 and heat is exchanged in a third heat exchanger 53 to liquefy water vapor. Other configurations remain consistent with those in the first embodiment, and the hydrocarbon generation system 1 of the present embodiment also achieves the same outcomes as in the first embodiment.

(Third Embodiment)

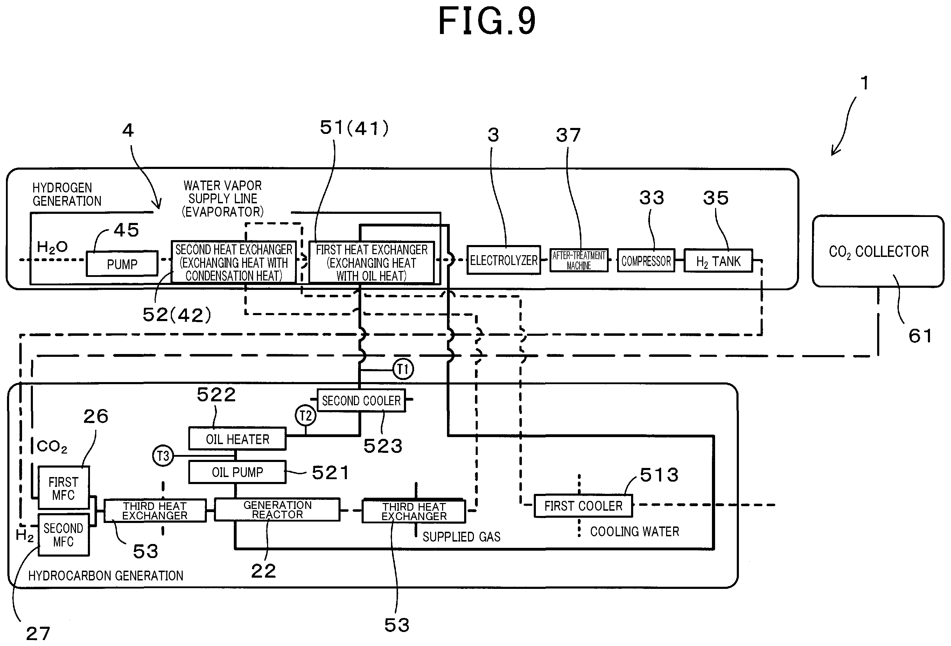

The present embodiment describes an embodiment of a hydrocarbon generation system having a second heat exchanger, with reference to FIGs 9 to 16. As shown in FIG. 9, a second heat exchanger 52 exchanges heat by utilizing thermal energy derived from the generated gas containing water vapor emitted by a hydrocarbon generator 2, applying it to heat either liquid water or water vapor of raw materials in a water vapor supply line 4.

In the present embodiment, the water vapor supply line 4 has, for example, evaporators 41 and 42 (specifically, first and second evaporators 41 and 42) and a pump 45. The evaporators 41 and 42 apply thermal energy to heat the liquid water and/or water vapor of the raw materials. The first evaporator 41 is configured to exchange heat with a first heat exchanger 51 using heat transfer oil. The second evaporator 42 is configured to exchange heat with the second heat exchanger 52 by the generated gas.

As described above, the second heat exchanger 52 exchanges heat by the thermal energy derived from the generated gas containing the water vapor emitted by the hydrocarbon generator 2, applying it to heat either the liquid water or water vapor of the raw materials in the water vapor supply line 4. The heat of the generated gas corresponds to the thermal energy retained in the generated gas emitted from a generation reactor 22 of the hydrocarbon generator 2. Therefore, the heat of the generated gas differs from the heat of the reaction generated through the hydrocarbon generation reaction in the generation reactor 22. In an emission path of the generated gas, downstream of the first heat exchanger 51, a first cooler 513 can be provided to further cool the generated gas exchanged heat in the first heat exchanger 51. Note that a refrigerant used in the first cooler 513 is water, for example.

The second heat exchanger 52 recovers the thermal energy from the generated gas (specifically, hydrocarbons such as methane and water vapor) emitted from the hydrocarbon generator 2 and applies the recovered thermal energy to heat the liquid water or water vapor as the raw materials. The second heat exchanger 52 exchanges heat by utilizing at least water vapor (i.e., steam) in the generated gas as a heat exchange medium, applying heat of this water vapor to heat the liquid water or water vapor of the raw materials. In this case, the second heat exchanger 52 can use condensation heat of the water vapor to evaporate water in the water vapor supply line 4. Furthermore, when the second heat exchanger 52 exchanges heat using heat of hydrocarbon, a heat balance in the hydrocarbon generation system 1 is further improved, resulting in greater thermal efficiency. The heat of the reaction and latent heat from evaporation occurs due to the hydrocarbon generation reaction. The latent heat from evaporation is distributed to water vapor of the generated gas, and the heat of the reaction is distributed to the heat transfer oil and/or generated gas. Thus, the heat of the generated gas is composed of the latent heat for evaporation and some of the heat of the reaction. The heat of the generated gas may also be referred to as heat retained in the generated gas after being recovered by the heat transfer oil in the first heat exchanger 51.

As shown in FIG. 9, the water vapor supply line 4 may have the first evaporator 41 that exchanges heat with the first heat exchanger 51 by using heat retained in the generated gas and the second evaporator 42 that exchanges heat with the second heat exchanger 52 by using the heat of the reaction generated through the hydrocarbon generation reaction. One evaporator may exchange heat with the first and second heat exchangers 51 and 52 instead of the first and second evaporators 41 and 42.

As shown in FIG. 9, the first heat exchanger 51 is preferably disposed downstream of the second heat exchanger 52 in a gas flow path. In this case, stable heat exchange is possible even if an amount of thermal energy recovered in the first heat exchanger 51 fluctuates. This is because the heat transfer oil, which possesses a high heat capacity, is used in the first heat exchanger 51, disposed downstream of the second heat exchanger 52, facilitating efficient heat exchange in the first heat exchanger 51.

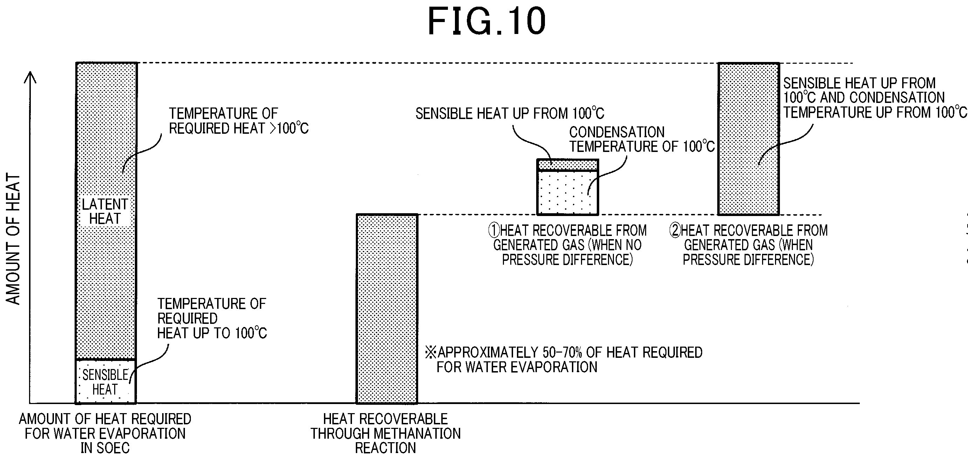

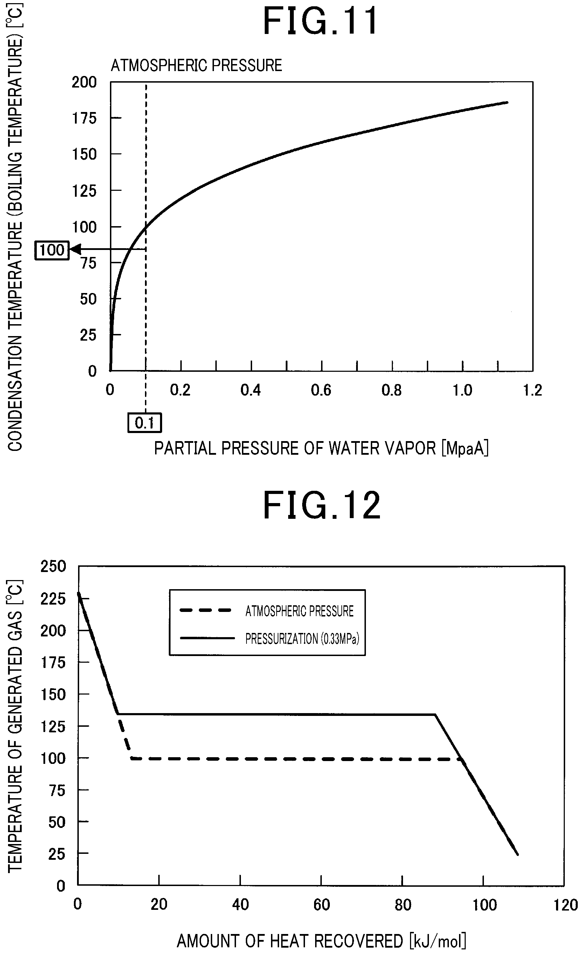

As shown in FIG. 10, the generation of the water vapor of the raw materials in the water vapor supply line 4 typically requires sensible heat to raise a temperature of the liquid water of the raw materials to 100°C, as well as latent heat associated with phase transition beyond 100°C. The heat of the reaction in the hydrocarbon generation reaction can usually cover approximately 50 to 70% of heat required for water evaporation. On the other hand, a condensation temperature (boiling temperature) of water depends on a partial pressure of water vapor. As shown in FIG. 11, the condensation temperature is 100°C at the atmospheric pressure (about 0.1 MPa). However, by occurring the hydrocarbon generation reaction under high pressure, the partial pressure of water vapor also increases, allowing for a higher condensation temperature. This increases an amount of heat recovered from the generated gas. Furthermore, the recovered heat and the heat of the reaction generated through the hydrocarbon generation reaction can provide all the thermal energy required to generate the water vapor of the raw materials (see FIG. 10).

In the hydrocarbon generation system 1, a partial pressure P1 of water vapor of the generated gas in the second heat exchanger 52 is preferably higher than a pressure P2 of the water vapor of the raw materials supplied to an electrolyzer 3. The partial pressure P1 of the water vapor in the generated gas can be increased, for example, by compressing hydrogen and/or carbon dioxide that flows into the hydrocarbon generator 2. The compression of hydrogen is performed by a compressor 33 that compresses hydrogen generated in the electrolyzer 3, for example, as shown in FIG. 9. Although a configuration is not shown in the figure, a $CO_2$ compressor is provided, for example, to compress carbon dioxide, which pressurizes carbon dioxide supplied to the hydrocarbon generator 2. Accordingly, the partial pressure P1 of the water vapor also becomes high. Thus, by compressing hydrogen and/or carbon dioxide, the generated gas is pressurized, and the partial pressure of the water vapor P1 can be increased. Preferably, hydrogen may be compressed and pressurized by the compressor 33 disposed downstream of the electrolyzer 3 in the gas flow path. In this case, the partial pressure P1 of the water vapor in the generated gas can be increased more efficiently than the pressure P2 of the water vapor of the raw materials. This is because the hydrogen generated in the electrolyzer 3 is under low pressure.

When the generated gas is not pressurized (specifically, when the pressure of the generated gas is the same as the atmospheric pressure), a condensation temperature of water vapor is 100°C. Therefore, condensation heat, which constitutes 70 to 80% of the heat of the generated gas, may no longer be available for latent heat of evaporation but can be used for sensible heat. In contrast, when the generated gas is pressurized (specifically, when the pressure of the generated gas is higher than the atmospheric pressure), the partial pressure P1 of the water vapor in the generated gas is higher than the pressure P2 of the water vapor of the raw materials. Therefore, the condensation heat can also be used for the latent heat of evaporation. This increases the amount of heat recovered and further improves the heat balance in hydrocarbon generation system 1. When the generated gas is pressurized, a pressure in, for example, the production reactor 22 of the hydrocarbon generator 2 is typically higher. Therefore, a reaction rate of hydrocarbon generation increases.

To enhance the heat balance in the hydrocarbon generation system 1, the second heat exchanger 52 is configured to evaporate some or all the liquid water of the raw materials heated within the second heat exchanger 52. In other words, the system may preferably be configured to use the condensation heat of the water vapor in the generated gas as the latent heat of evaporation of the liquid water of the raw materials. Specific configurations for using condensation heat of water vapor to the liquid water of the raw materials include compressors for compressing hydrogen and/or carbon dioxide, as described above.

The following equations (VII) to (IX) show major chemical reactions in the hydrocarbon generation system 1.

$$H_2O \text{ (liquid: 25°C)} \rightarrow H_2O \text{ (gas: 100°C)} \ (\Delta H = 46.3 \text{ kJ/mol}) \ ... \quad \text{(VII)}$$

$$H_2O \rightarrow H_2 + 1/2O_2 \ ... \quad \text{(VIII)}$$

$$CO_2 + 4H_2 \rightarrow CH_4 + 2H_2O \ (\Delta H = -165 \text{ kJ/mol}) \ ... \quad \text{(IX)}$$

As shown in the equations (VII) to (IX), for example, to synthesize $CH_4$ at a rate of 1 mol/sec, water must evaporate at a rate of 4 mol/sec. An amount of heat generated when $CH_4$ is synthesized at a rate of 1 mol/sec is 165 kW (= 165 kJ/sec). On the other hand, heat required for evaporation of water is 185 kW (= 46.3 × 4 kW = 185 kJ/sec). Therefore, the heat of the reaction of the hydrocarbon generation reaction (specifically, methanation) alone cannot cover all the heat required for evaporation of water. Furthermore, due to heat dissipation, recovering all the generated heat is challenging. An amount of heat that can actually be recovered is approximately 70% of the 165 kW of heat generated in the hydrocarbon production reaction.

Since water vapor is generated in the hydrocarbon generation reaction, condensation heat of the water vapor can also be used. The condensation heat of the water vapor generated in methanation is approximately 80 kW in synthesis of methane at a rate of 1 mol/sec, depending on a pressure. However, if no difference is made between a pressure of the water vapor generated through methanation and a pressure of the water vapor of the raw materials supplied to the electrolyzer 3, the condensation temperature of the water vapor generated through methanation and the evaporation temperature of the water supplied to the electrolyzer 3 are approximately identical. Thus, temperature differences do not occur. A heat exchange volume Q in a heat exchanger can be defined by the following equation (X). If no temperature difference occurs (i.e., ΔT = 0), heat is not exchanged even when a heat exchange area is large. Note that, in the equation (X), 'A' indicates the heat exchange area, 'U' indicates an overall heat transfer coefficient, and "ΔT" indicates a logarithmic mean temperature difference.

$$Q = A \times U \times \Delta T \ ... \ (X)$$

In other words, the condensation heat of the water vapor generated through methanation can be used to preheat water supplied to the electrolyzer 3 to its boiling temperature but not to heat the evaporation of water. Consequently, the amount of heat that can be recovered is approximately 20% of the total heat of condensation.

In contrast, by pressurizing the generated gas, the condensation temperature of the water vapor in gas generated through methanation becomes higher than the evaporation temperature of the water supplied to the electrolyzer 3. Therefore, the condensation heat of the water vapor in the gas generated through methanation can be recovered as the heat required for evaporation of water supplied to the electrolyzer 3.

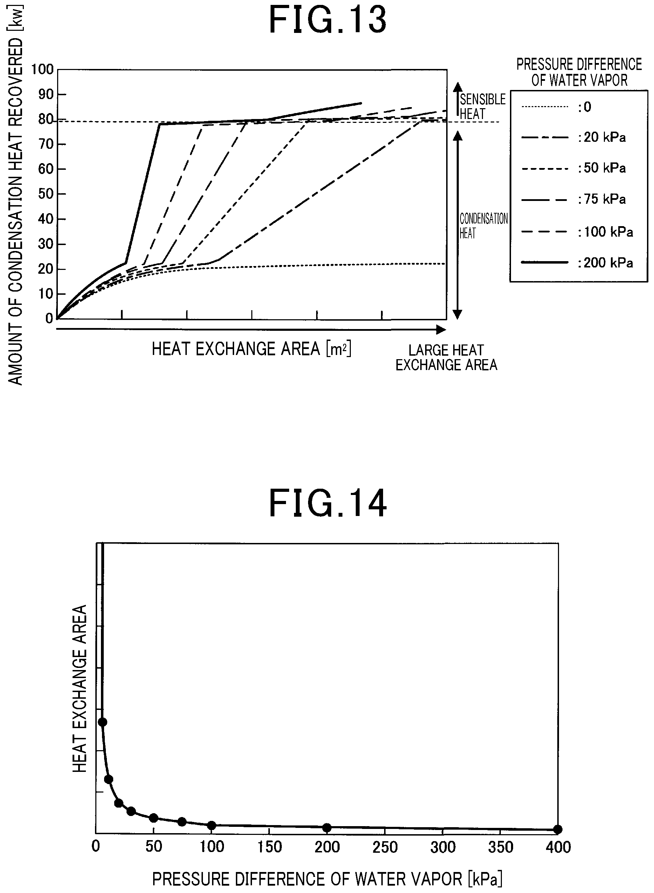

FIG. 13 is a graph showing a relationship between a heat exchange area of the second heat exchanger 52 and an amount of condensation heat recovered. A vertical axis in FIG. 13 indicates an amount of heat that can be recovered from water vapor when methane is synthesized at a rate of 1 mol/sec. Plots of 80 kW or more in FIG. 13 refer to an amount of heat recovered from liquid water after condensation.

As shown in FIG. 13, when a pressure difference of the water vapor is zero, increasing the heat exchange area allows for heat exchange of only approximately 20% of the condensation heat of the water vapor. On the other hand, as the pressure difference of the water vapor increases, a temperature difference between the condensation and evaporation temperatures also increases, resulting in a larger heat exchange volume per unit heat exchange area, for example. Consequently, the second heat exchanger 52 can be downsized.

FIG. 14 is a graph showing a relationship between a pressure difference of water vapor and a heat exchange area required to recover all condensation heat. As shown in FIG. 14, when the pressure difference is 50 kPa or more, the change in the required heat exchange area (a decreasing ratio) remains small, even with an increase in pressure. Then, by establishing a pressure difference (P1-P2) between the partial pressure P1 of the water vapor in the generated gas in the second heat exchanger 52 and the pressure P2 of the water vapor of the raw materials supplied to the electrolyzer 3 at 50 kPa or more, the heat exchange volume per unit heat exchange area can be significantly increased. FIGs. 13 and 14 both plot data calculated at a pressure of 100 kPa (equivalent to the atmospheric pressure) of the water vapor in the electrolyzer 3. The heat exchange area indicates the same trend as in FIGs. 13 and 14, even when a pressure of water vapor in a reference of the electrolyzer 3 is changed. To achieve pressure resistance of a heat exchanger, the pressure difference (P1-P2) may preferably be 1000 kPa or less.

The hydrocarbon generation system 1 may preferably be configured so that part of the heat from the generated gas emitted from the hydrocarbon generator 2 is used to heat carbon oxide and hydrogen supplied to the hydrocarbon generator 2. In this case, excess thermal energy of the generated gas that is not used in the water vapor supply line 4 can be utilized in the hydrocarbon production reaction. This achieves an excellent heat balance. A specific configuration for using some of the heat of the generated gas to heat carbon oxide and hydrogen is a heat exchanger (specifically, the third heat exchanger 53), as shown in FIG. 9. The third heat exchanger 53 recovers some of the heat from the generated gas and applies the recovered heat to a carbon oxide gas and hydrogen. For convenience of explanation, FIG. 9 shows two third heat exchangers, but they actually represent a single heat exchanger. Other configurations remain consistent with those in the first embodiment, and a hydrocarbon generation system 1 of the present embodiment also achieves the same outcomes as in the first embodiment.

Flowcharts shown in FIGs. 4, 7, 15, and 16 illustrate processes of the hydrocarbon generation system 1 of the present embodiment. The flowcharts are an example of controlling a temperature of the heat transfer oil to be in a range of 240 to 260°C. This temperature may be changed. For example, each process shown in the flowcharts is controlled by a control device that is not shown. Specifically, the control device, which includes one or more processors or the like, controls each process by the processor executing a program stored in a memory.

<Startup Process>

A startup process is the same as in the first embodiment, for example (see FIG. 4).

<Control of Hydrogen Generation>

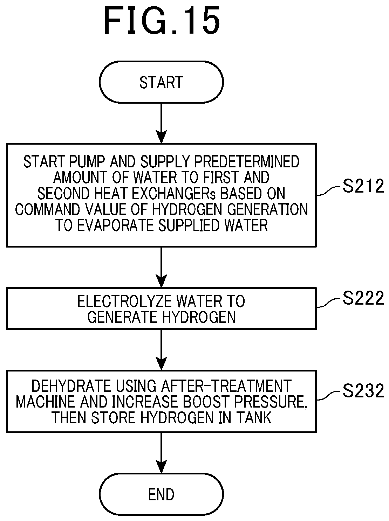

As shown in FIG. 15, when a hydrogen-generating process starts, a pump 45 of the water vapor supply line 4 is activated (S212). The pump 45 discharges a predetermined amount of water (specifically, liquid water of raw materials) to the first and second heat exchangers 51 and 52 based on a command value that defines an amount of hydrogen generated in response to the requirement. In the first and second heat exchangers 51 and 52, water evaporates to generate water vapor of raw materials (i.e., steam), and then the generated water vapor is emitted to the electrolyzer 3. In the electrolysis unit 3, water included in the water vapor is electrolyzed to generate hydrogen (S222). The generated hydrogen is dehydrated by an after-treatment machine 37, compressed by the compressor 33 to increase boost pressure, and stored in a $H_2$ tank 35 (S232) (for example, see FIG. 9).

<Control of Hydrocarbon Generation>

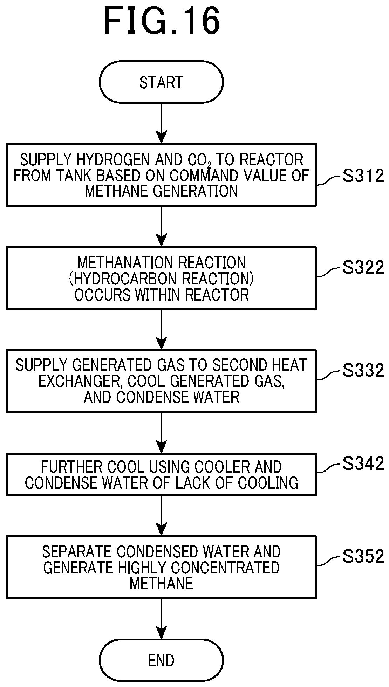

As shown in FIG. 16, when a hydrocarbon-generating process starts, hydrogen and $CO_2$ are supplied to the generation reactor 22 (S312) based on a command value that defines an amount of hydrocarbon generated in response to the requirement (e.g., an amount of methane generated in response to the requirement). The hydrogen is supplied from the $H_2$ tank 35 and $CO_2$ is supplied from a $CO_2$ collector 61. In the generation reactor 22, for example, a methanation reaction occurs to generate a gas containing methane and water (i.e., steam) (S322). The generated gas is supplied to the second heat exchanger 52 to cool and condense the generated water (S332). The generated water, which lacks cooling, is further cooled by the first cooler 513, which condenses it (S342). For example, the condensed water is separated by a gas-liquid separator 65 to generate highly concentrated methane (S352).

<Temperature Control for Heat Transfer Oil>

A temperature control process for the heat transfer oil is the same as in the first embodiment, for example (see FIG. 7).
In the hydrocarbon generation system 1 of the present embodiment, the first heat exchanger 51 exchanges heat and applies the heat of the heat transfer oil to the liquid water of the raw materials in the water vapor supply line 4. Furthermore, the second heat exchanger 52 exchanges heat from the generated gas with the liquid water or water vapor of the raw materials in the water vapor supply line 4, for example, to apply the heat to the water vapor of the raw materials. This enhances thermal efficiency of the hydrocarbon generation system 1.

(Forth Embodiment)

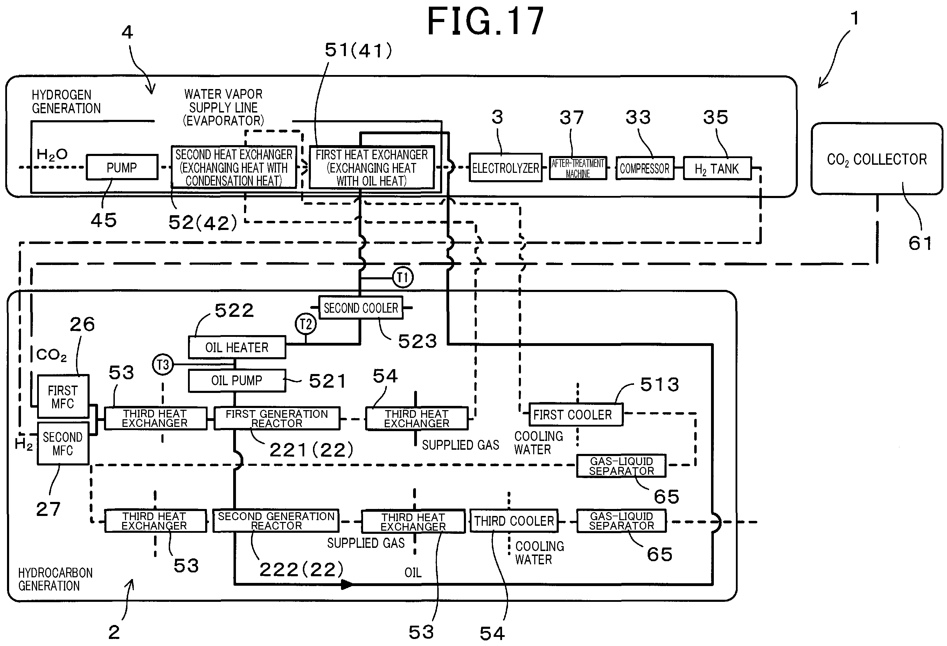

The present embodiment describes an embodiment of a hydrocarbon generation system having two hydrocarbon generators, with reference to FIG 17. As shown in FIG. 17, a hydrocarbon generation system 1 of the present embodiment has two hydrocarbon generators 2, which are connected in series, as in the second embodiment. Other configurations remain consistent with those in the third embodiment.
A second heat exchanger 52 exchanges heat of the generated gas emitted from each hydrocarbon generator 2 with liquid water or water vapor of raw materials in a water vapor supply line 4. Preferably, the second heat exchanger 52 exchanges heat of the generated gas emitted from the hydrocarbon generator 2 (specifically, a first generation reactor 221), which is disposed most upstream in a gas path, with the liquid water or water vapor of the raw materials in the water vapor supply line 4. In this case, thermal efficiency of the hydrocarbon generation system 1 is further enhanced. A reaction rate of hydrocarbon generation is highest in the first generation reactor 221. This is because the water vapor is a most abundant component in the generated gas. Other configurations remain consistent with those in the third embodiment, and the hydrocarbon generation system 1 of the present embodiment also achieves the same outcomes as in the third embodiment.

(Fifth Embodiment)

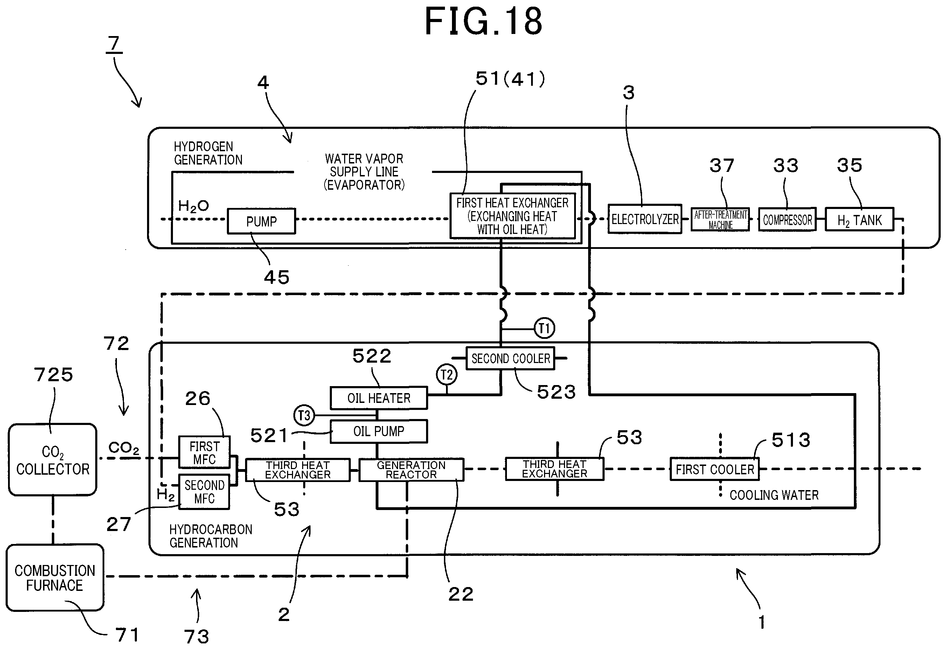

The present embodiment describes an embodiment of a hydrocarbon generation system with reference to FIG 18. As shown in FIG.18, a carbon dioxide circulation system 7 of the present embodiment has a hydrocarbon generation system 1, a combustion furnace 71, a carbon dioxide supply line 72, and a hydrocarbon supply line 73. For example, the hydrocarbon generation system 1 may be similar to the first embodiment, as shown in FIG. 18. The configuration may also be similar to that of any of the second through fourth embodiments.

The combustion furnace 71 is a facility that uses hydrocarbon as fuel, generating carbon dioxide as a result of combustion. The combustion furnace 71 includes, for example, incinerators provided in factories and various types of melting furnaces, such as aluminum melting furnaces. The combustion furnace 71 emits carbon dioxide.

The carbon dioxide supply line 72 supplies carbon dioxide emitted from the combustion furnace 71 to a hydrocarbon generator 2 of the hydrocarbon generation system 1. In the hydrocarbon generator 2, carbon dioxide is used as a carbon oxide gas, and hydrocarbon is generated through the hydrocarbon generation reaction.

The hydrocarbon supply line 73 supplies the hydrocarbon generated by the hydrocarbon generator 2 to the combustion furnace 71. In the combustion furnace 71, the generated hydrocarbon is used as fuel and burned, as described above. Thus, in the carbon dioxide circulation system 7, carbon dioxide circulates between the hydrocarbon generation system 1 and the combustion furnace 71. This makes it possible to construct a so-called carbon-neutral system capable of a reduction of greenhouse gas emissions.

The carbon dioxide circulation system 7 is configured to adjust an amount of the hydrocarbon generated by the hydrocarbon generator 2 depending on an operating status of the combustion furnace 71. Adjustment of the amount of the generated hydrocarbon is controlled by a control device that is not shown. This allows the hydrocarbon generation system 1 to generate and supply a requirement amount of hydrocarbon to the combustion furnace 71, even if the requirement amount thereof fluctuates depending on the operating status of the combustion furnace 71. Adjustment of an amount of the supply is achieved, for example, by adjusting the amount of hydrogen and hydrocarbon generations in the hydrocarbon generation system 1.

In the carbon dioxide circulation system 7, for system simplification, it is preferable that all hydrogen supplied to the hydrocarbon generator 2 is supplied from an electrolyzer 3. In this case, the heat used for water evaporation in a water vapor supply line 4 is, on average, more significant than the heat of the reaction generated through the hydrocarbon reaction. Therefore, a supply of thermal energy is necessary; however, in the present embodiment, a first heat exchanger 51 enables the additional supply of thermal energy from heat transfer oil.

The present disclosure is not limited to the above embodiments but may be applied to various embodiments as long as it does not depart from the essence of the disclosure.

Technical features of the present disclosure are as follows.

[1] A hydrocarbon generation system including:

a hydrocarbon generator (2, 21, 22) that generates hydrocarbon through an exothermic reaction between a carbon oxide gas and hydrogen;
an electrolyzer (3) that generates hydrogen from water vapor of raw materials, the generated hydrogen being supplied to the hydrocarbon generator;
a water vapor supply line (4) that generates the water vapor of the raw materials by evaporating liquid water of the raw materials and supplies the generated water vapor to the electrolyzer;
a heat exchanger (51) that uses heat of a reaction generated in the hydrocarbon generator to evaporate the liquid water of the raw materials in the water vapor supply line via heat transfer oil.

[2] The hydrocarbon generation system according to [1], in which
no water evaporation means is provided between the heat exchanger and the electrolyzer in the water vapor supply line.

[3] The hydrocarbon generation system according to [1] or [2], further including a heater that heats the heat transfer oil and a temperature sensor that measures a temperature of the heat transfer oil flowing into the heater, in which
the heater is disposed downstream of the heat exchanger in a circulation path of the heat transfer oil, and is configured to control an amount of heating of the heat transfer oil based on a measurement value of the temperature sensor.

[4] The hydrocarbon generation system according to [3], in which
the heater is configured to warm up the hydrocarbon generator at a startup.

[5] The hydrocarbon generation system according to any one of [1] to [4], in which

the heat exchanger corresponds to a first heat exchanger, and
the hydrocarbon generation system further includes a second heat exchanger that exchanges heat from a generated gas containing the water vapor emitted from the hydrocarbon generator with the liquid water or water vapor of the raw materials in the water vapor supply line.

[6] The hydrocarbon generation system according to [5], in which
a partial pressure P1 of the water vapor of the generated gas in the second heat exchanger is higher than a pressure P2 of the water vapor of the raw materials supplied to the electrolyzer.
[7] The hydrocarbon generation system according to any of [1] to [6], further including a buffer tank (35) for storing hydrogen, which is disposed between the hydrocarbon generator and the electrolyzer.
[8] A carbon dioxide circulation system (7) including:
the hydrocarbon generation system (1) according to any one of [1] to [7];

a combustion furnace (71) that uses hydrocarbon as fuel and emits carbon dioxide;
a carbon dioxide supply line (72) that supplies carbon dioxide emitted from the combustion furnace to the hydrocarbon generator in the hydrocarbon generation system as the carbon oxide gas; and
a hydrocarbon supply line (73) that supplies hydrocarbon generated by the hydrocarbon generator to the combustion furnace, in which
the carbon dioxide circulation system is configured to adjust an amount of hydrocarbon generated by the hydrocarbon generator depending on an operating status of the combustion furnace.

Although the present disclosure has been described in accordance with the embodiments, this disclosure is not limited to the above embodiments or structures. The present disclosure also includes various variants and variations within the scope of equality. Additionally, various combinations and embodiments, along with other combinations and embodiments that include one element, more or less, fall within the scope and concept of this disclosure. Although this disclosure has been described in accordance with an embodiment, this disclosure is not limited to said embodiment or structure.

**Claims**

1. 1. A hydrocarbon generation system comprising:

a hydrocarbon generator (2, 21, 22) that generates hydrocarbon through an exothermic reaction between a carbon oxide gas and hydrogen;
an electrolyzer (3) that generates hydrogen from water vapor of raw materials, the generated hydrogen being supplied to the hydrocarbon generator;
a water vapor supply line (4) that generates the water vapor of the raw materials by evaporating liquid water of the raw materials and supplies the generated water vapor to the electrolyzer;
a heat exchanger (51) that uses heat of a reaction generated in the hydrocarbon generator to evaporate the liquid water of the raw materials in the water vapor supply line via heat transfer oil.

2. 2. The hydrocarbon generation system according to claim 1, wherein
no water evaporation means is provided between the heat exchanger and the electrolyzer in the water vapor supply line.

3. 3. The hydrocarbon generation system according to claim 1 or 2, further comprising a heater that heats the heat transfer oil and a temperature sensor that measures a temperature of the heat transfer oil flowing into the heater, wherein
the heater is disposed downstream of the heat exchanger in a circulation path of the heat transfer oil, and is configured to control an amount of heating of the heat transfer oil based on a measurement value of the temperature sensor.

4. 4. The hydrocarbon generation system according to claim 3, wherein
the heater is configured to warm up the hydrocarbon generator at a startup.

5. 5. The hydrocarbon generation system according to claim 3, wherein

the heat exchanger corresponds to a first heat exchanger, and
the hydrocarbon generation system further comprises a second heat exchanger that exchanges heat from a generated gas containing the water vapor emitted from the hydrocarbon generator with the liquid water or water vapor of the raw materials in the water vapor supply line.

6. 6. The hydrocarbon generation system according to claim 5, wherein
a partial pressure P1 of the water vapor of the generated gas in the second heat exchanger is higher than a pressure P2 of the water vapor of the raw materials supplied to the electrolyzer.

7. 7. The hydrocarbon generation system according to claim 1 or 2, further comprising a buffer tank (35) for storing hydrogen, which is disposed between the hydrocarbon generator and the electrolyzer.

8. 8. A carbon dioxide circulation system (7) comprising:

the hydrocarbon generation system (1) according to claim 1 or 2;
a combustion furnace (71) that uses hydrocarbon as fuel and emits carbon dioxide;
a carbon dioxide supply line (72) that supplies carbon dioxide emitted from the combustion furnace to the hydrocarbon generator in the hydrocarbon generation system as the carbon oxide gas; and
a hydrocarbon supply line (73) that supplies hydrocarbon generated by the hydrocarbon generator to the combustion furnace, wherein
the carbon dioxide circulation system is configured to adjust an amount of hydrocarbon generated by the hydrocarbon generator depending on an operating status of the combustion furnace.

# FIG.1

51(41)

4

CIRCULATION OF HEAT
EXCHANGE MEDIUM (OIL)

3

1

$H_2O$ — PUMP — FIRST HEAT EXCHANGER — ELECTROLYZER

45

T1 — 230 ~260°C

523 — COOLER

250~260°C

T2

522 — OIL HEATER

$H_2$ TANK — 35

T3 — 250°C EXOTHERMIC REACTION

$CH_4 + 2H_2O$ ← HYDROCARBON GENERATOR

$4H_2$

$CO_2$

2

FIG.2

61 CO₂ COLLECTOR

1

HYDROGEN GENERATION

3 ELECTROLYZER
37 AFTER-TREATMENT MACHINE
33 COMPRESSOR
35 H₂ TANK

WATER VAPOR SUPPLY LINE (EVAPORATOR)

H₂O
45 PUMP

51(41) FIRST HEAT EXCHANGER (EXCHANGING HEAT WITH OIL HEAT)

T1
523 SECOND COOLER
T2
522 OIL HEATER
T3
521 OIL PUMP

513 FIRST COOLER
COOLING WATER

53 THIRD HEAT EXCHANGER

22 GENERATION REACTOR

26 FIRST MFC
27 SECOND MFC

CO₂
H₂

53 THIRD HEAT EXCHANGER

2 HYDROCARBON GENERATION

FIG.3

# FIG.4

```
           ┌──────────────┐
           │    START     │
           └──────┬───────┘
                  ▼
     ┌────────────────────────┐
     │ START HEAT TRANSFER OIL │─── S11
     │ PUMP TO CIRCULATE HEAT  │
     │     TRANSFER OIL        │
     └────────────┬───────────┘
                  ▼◄──────────────┐
     ┌────────────────────────┐   │
     │ HEAT HEAT TRANSFER OIL │── S12
     │       BY HEATER        │   │
     └────────────┬───────────┘   │
                  ▼    S13         │
              ╱─────────╲      NO  │
             ╱ T2 > 250°C ╲───────┘
              ╲─────────╱
                  │ YES
                  ▼
     ┌────────────────────────┐
     │      STOP HEATER       │── S14
     └────────────┬───────────┘
                  ▼
     ┌────────────────────────┐
     │   COMPLETION OF        │── S15
     │   STARTUP PROCESS      │
     └────────────────────────┘
```

# FIG.5

START

↓

START PUMP AND SUPPLY PREDETERMINED
AMOUNT OF WATER TO FIRST HEAT
EXCHANGER BASED ON COMMAND
VALUE OF HYDROGEN GENERATION TO
EVAPORATE SUPPLIED WATER — S211

↓

ELECTROLYZE WATER TO
GENERATE HYDROGEN — S221

↓

DEHYDRATE USING AFTER-TREATMENT
MACHINE AND INCREASE BOOST PRESSURE,
THEN STORE HYDROGEN IN TANK — S231

↓

END

# FIG.6

START

↓

SUPPLY HYDROGEN AND $CO_2$ TO REACTOR
FROM TANK BASED ON COMMAND VALUE OF
METHANE GENERATION — S311

↓

METHANATION REACTION
(HYDROCARBON REACTION)
OCCURS WITHIN REACTOR — S321

↓

COOL GENERATED GAS USING COOLER
AND CONDENSE WATER — S331

↓

SEPARATE CONDENSED WATER AND
GENERATE HIGHLY CONCENTRATED
METHANE — S341

↓

END

# FIG.7

START

↓

START HEAT TRANSFER OIL PUMP TO CIRCULATE HEAT TRANSFER OIL — S41

↓

S42

T1 > 260°C — NO

YES ↓

S43

COOL HEAT TRANSFER OIL USING COOLER — S43

↓

S44

NO — T2 < 250°C

YES ↓

STOP COOLER — S45

↓

S46

NO — T2 < 240°C

YES ↓

HEAT HEAT TRANSFER OIL BY HEATER — S47

↓

S48

NO — T3 > 250°C

YES ↓

STOP HEATER — S49

# FIG.8

EP 4 600 236 A1

# FIG.9

# FIG.10

# FIG.11

X-axis: PARTIAL PRESSURE OF WATER VAPOR [MpaA]
Y-axis: CONDENSATION TEMPERATURE (BOILING TEMPERATURE) [°C]
ATMOSPHERIC PRESSURE

# FIG.12

X-axis: AMOUNT OF HEAT RECOVERED [kJ/mol]
Y-axis: TEMPERATURE OF GENERATED GAS [°C]

Legend:
- - - - ATMOSPHERIC PRESSURE
——— PRESSURIZATION (0.33MPa)

# FIG.13

Y-axis: AMOUNT OF CONDENSATION HEAT RECOVERED [kw]
X-axis: HEAT EXCHANGE AREA [m²] — LARGE HEAT EXCHANGE AREA

SENSIBLE HEAT
CONDENSATION HEAT

PRESSURE DIFFERENCE OF WATER VAPOR
: 0
: 20 kPa
: 50 kPa
: 75 kPa
: 100 kPa
: 200 kPa

# FIG.14

Y-axis: HEAT EXCHANGE AREA
X-axis: PRESSURE DIFFERENCE OF WATER VAPOR [kPa]

# FIG.15

START

START PUMP AND SUPPLY PREDETERMINED
AMOUNT OF WATER TO FIRST AND
SECOND HEAT EXCHANGERs BASED ON
COMMAND VALUE OF HYDROGEN GENERATION
TO EVAPORATE SUPPLIED WATER — S212

ELECTROLYZE WATER TO
GENERATE HYDROGEN — S222

DEHYDRATE USING AFTER-TREATMENT
MACHINE AND INCREASE BOOST PRESSURE,
THEN STORE HYDROGEN IN TANK — S232

END

# FIG.16

```
        ┌─────────────┐
        │    START    │
        └──────┬──────┘
               │
               ▼
┌──────────────────────────────────┐
│ SUPPLY HYDROGEN AND CO₂ TO REACTOR│
│ FROM TANK BASED ON COMMAND VALUE OF├── S312
│      METHANE GENERATION           │
└──────────────┬───────────────────┘
               │
               ▼
┌──────────────────────────────────┐
│      METHANATION REACTION         │
│     (HYDROCARBON REACTION)        ├── S322
│       OCCURS WITHIN REACTOR       │
└──────────────┬───────────────────┘
               │
               ▼
┌──────────────────────────────────┐
│ SUPPLY GENERATED GAS TO SECOND HEAT│
│ EXCHANGER, COOL GENERATED GAS,    ├── S332
│      AND CONDENSE WATER           │
└──────────────┬───────────────────┘
               │
               ▼
┌──────────────────────────────────┐
│   FURTHER COOL USING COOLER AND   ├── S342
│  CONDENSE WATER OF LACK OF COOLING │
└──────────────┬───────────────────┘
               │
               ▼
┌──────────────────────────────────┐
│   SEPARATE CONDENSED WATER AND    │
│   GENERATE HIGHLY CONCENTRATED    ├── S352
│            METHANE                │
└──────────────┬───────────────────┘
               │
               ▼
        ┌─────────────┐
        │     END     │
        └─────────────┘
```

FIG.17

# FIG.18

EP 4 600 236 A1

## INTERNATIONAL SEARCH REPORT

| International application No. |
| --- |
| **PCT/JP2023/036113** |

| A. | CLASSIFICATION OF SUBJECT MATTER |
| --- | --- |

*C07C 1/12*(2006.01)i; *C07C 1/04*(2006.01)i; *C07C 9/04*(2006.01)i
FI:    C07C1/12; C07C9/04; C07C1/04

According to International Patent Classification (IPC) or to both national classification and IPC

| B. | FIELDS SEARCHED |
| --- | --- |

Minimum documentation searched (classification system followed by classification symbols)

    C07C1/12; C07C1/04; C07C9/04

Documentation searched other than minimum documentation to the extent that such documents are included in the fields searched

    Published examined utility model applications of Japan 1922-1996
    Published unexamined utility model applications of Japan 1971-2023
    Registered utility model specifications of Japan 1996-2023
    Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2015-513531 A (THERMOGAS DYNAMICS LIMITED) 14 May 2015 (2015-05-14) claims 12, 24, 26-27, 74, paragraphs [0018], [0046] | 1-8 |
| Y | JP 2020-093216 A (IHI CORP) 18 June 2020 (2020-06-18) paragraphs [0037], [0038] | 1-8 |
| A | US 2015/0080483 A1 (SIEMENS AKTIENGESELLSCHAFT) 19 March 2015 (2015-03-19) entire text | 1-8 |

☐ Further documents are listed in the continuation of Box C.   ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **11 December 2023** | **19 December 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/036113**

| Patent document cited in search report | Publication date (day/month/year) | Patent family member(s) | Publication date (day/month/year) |
|---|---|---|---|
| JP 2015-513531 A | 14 May 2015 | US 2016/0017800 A1<br>claims 12, 24, 26, 74,<br>paragraphs [0023], [0055]<br>WO 2013/124632 A2<br>EP 2817438 A2<br>CN 104271807 A | |
| JP 2020-093216 A | 18 June 2020 | (Family: none) | |
| US 2015/0080483 A1 | 19 March 2015 | WO 2013/152942 A1<br><br>EP 2650401 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022161211 A

- JP 2019108238 A